# FASCICULE DE BREVET EUROPEEN

(11) **EP 3 999 155 B1**
(45) Date de publication et mention de la délivrance du brevet: **06.09.2023**
(21) Numéro de dépôt: 20753988.3
(22) Date de dépôt: 16.07.2020
(51) Int. Cl.: A61M 15/08, B05B 11/00, B05B 11/10, B05B 12/12

(54) **DISPOSITIF DE DISTRIBUTION NASALE DE PRODUIT FLUIDE**
VORRICHTUNG ZUR NASALEN VERABREICHUNG EINES FLÜSSIGPRODUKTS
DEVICE FOR NASAL DELIVERY OF FLUID PRODUCT

(30) Priorité: 19.07.2019 FR 1908237
(43) Date de publication de la demande: 25.05.2022
(73) Titulaire: APTAR France SAS, 27110 Le Neubourg (FR)
(72) Inventeur: ADAM, Fabien, 27930 AVIRON (FR); FARROCO, Vincent, 76630 ENVERMEU (FR)
(74) Mandataire: CAPRI
(86) Numéro de dépôt international: PCT/FR2020/051286
(87) Numéro de publication internationale: WO 2021/014078

(56) Documents cités:
- WO-A1-2018/109409
- WO-A2-2014/165694

## Description

La présente invention concerne un dispositif nasal de distribution de produit fluide.

Les dispositifs de distribution nasale sont bien connus. Ils comportent généralement un réservoir contenant une ou plusieurs doses de produits fluide et une tête de distribution pour distribuer le produit fluide, notamment via une pompe, une valve doseuse ou un piston coulissant dans ledit réservoir. Lorsque l'utilisateur souhaite utiliser le dispositif, il insère la tête de distribution dans la narine et actionne le dispositif pour distribuer une dose de produit fluide, généralement sous forme de spray.

Un inconvénient avec les dispositifs de l'art antérieur concerne l'efficacité de la dose distribuée dans la narine, qui dépend souvent de l'orientation du dispositif au moment de la distribution de la dose. Par exemple, lorsque le produit fluide distribué a pour objectif d'agir sur le cerveau, seule une partie minime de la dose atteint généralement la zone cible pour ce type de traitement, à savoir la zone olfactive comprenant les éthmoïdes, notamment en raison d'une orientation du dispositif d'administration dans la narine qui est variable d'un patient à un autre. Or, il apparait que cette orientation conditionne la bonne visée de la zone cible, en particulier dans le cas d'un spray fermé utilisé pour obtenir le maximum de déposition dans la zone cible.

Le document WO9857690 décrit un dispositif de distribution nasale comportant un moyen d'orientation en appui sur la lèvre supérieure de l'utilisateur. S'il améliore la qualité de l'insertion, un tel dispositif d'orientation ne permet pas de garantir une orientation optimale au moment de la distribution de la dose.

Les documents WO02085282, FR3024655, WO2014165694 et WO2018109409 décrivent d'autres dispositifs de l'état de la technique.

La présente invention a pour but de fournir un dispositif de distribution qui ne reproduit pas les inconvénients susmentionnés.

La présente invention a notamment pour but de fournir un dispositif de distribution nasale permettant de maîtriser l'orientation du dispositif dans la narine, quelle que soit la morphologie du patient et quelle que soit sa position, debout, couché ou incliné, au moment de l'utilisation du dispositif.

La présente invention a aussi pour but de fournir un dispositif de distribution nasale qui améliore le taux de déposition de produit actif sur la zone olfactive et/ou les éthmoïdes.

La présente invention a également pour but de fournir un dispositif de distribution nasale qui permette de renseigner l'utilisateur en temps réel sur la qualité d'insertion du dispositif dans la narine.

La présente invention a encore pour but de fournir un dispositif de distribution nasale qui permette à l'utilisateur de corriger l'orientation du dispositif dans la narine au moment de son actionnement.

La présente invention a également pour but de fournir un ensemble de distribution nasale qui est simple et peu coûteux à fabriquer et à assembler.

La présente a donc pour objet un dispositif de distribution nasale de produit fluide, comportant un réservoir contenant du produit fluide, un organe de distribution pour distribuer une dose de produit fluide à chaque actionnement, et une tête de distribution assemblée sur ledit organe de distribution, ladite tête de distribution étant pourvue d'un orifice de distribution, ledit dispositif nasal comportant:
- une caméra pour déterminer le positionnement spatial dudit dispositif nasal dans une narine de l'utilisateur, ladite caméra déterminant l'angle d'inclinaison verticale α et l'angle d'inclinaison latérale β entre l'axe longitudinal X dudit dispositif nasal et un axe A du visage, ledit axe A étant identifié au moyen de plusieurs points de repère détectés par ladite caméra, notamment trois repères sur le nez, six sur chaque oeil et cinq sur chaque sourcil, l'orientation dudit axe A par rapport à ladite narine étant toujours identique quelle que soit la position de l'utilisateur,
- un indicateur de positionnement pour guider l'utilisateur vers une orientation optimale du dispositif nasal dans ladite narine, et
- un module électronique et des moyens d'actionnement automatique adaptés à actionner automatiquement ledit dispositif nasal lorsque ladite caméra détermine que ledit dispositif nasal est disposé dans une position optimale dans ladite narine.

Avantageusement, le dispositif nasal comporte un corps, comprenant un corps supérieur disposé autour dudit réservoir et dudit organe de distribution, et un corps inférieur, disposé sous ledit réservoir.

Avantageusement, ledit corps inférieur est amovible dudit corps supérieur.

Avantageusement, ledit corps inférieur contient lesdits moyens d'actionnement automatique, notamment un élément poussoir et un moteur, adaptés lors de l'actionnement à déplacer ledit réservoir axialement vers le haut par rapport à ladite tête de distribution, pour ainsi actionner ledit organe de distribution.

Avantageusement, ledit indicateur de positionnement est un indicateur visuel visible par l'utilisateur lorsque le dispositif nasal est inséré dans ladite narine.

Avantageusement, ledit indicateur comporte une tête d'indication lumineuse pourvue d'une pluralités d'indicateurs lumineux, tels que des diodes électroluminescentes, adaptées à assister et guider l'utilisateur en temps réel pour améliorer le positionnement dudit dispositif nasal.

Avantageusement, ladite caméra est disposée au centre desdits indicateurs lumineux.

Avantageusement, ledit indicateur de positionnement est un indicateur sonore et/ou tactile.

Avantageusement, ladite caméra et ledit indicateur sont déportés radialement dudit dispositif nasal, en étant notamment formés sur un bras solidaire, notamment de manière articulée, dudit dispositif nasal.

Avantageusement, ladite caméra détermine la profondeur d'insertion P du dispositif nasal dans la narine, notamment par détermination de l'écart en pixels entre les deux yeux qui varie en fonction de la profondeur d'insertion.

Avantageusement, ledit module électronique, tel qu'un circuit imprimé, comporte un microprocesseur contenant un logiciel de traitement des informations fournies par ladite caméra.

Avantageusement, le dispositif nasal comporte en outre un module de communication sans fil, avantageusement un module Bluetooth^{®}, pour une communication avec un appareil mobile distant.

Avantageusement, le dispositif nasal comprend un écran adapté à afficher des informations visibles par l'utilisateur.

Avantageusement, le dispositif nasal comprend un haut-parleur et/ou un élément vibrant, pour fournir une indication sonore et/ou tactile pour guider l'utilisateur lors du positionnement du dispositif nasal dans ladite narine.

Avantageusement, le dispositif comporte un capteur, tel qu'un accéléromètre, un gyroscope, un capteur de luminosité, un capteur infrarouge, un capteur d'humidité, un capteur de température, pour détecter l'insertion dudit dispositif nasal dans ladite narine.

Avantageusement, ledit réservoir contient plusieurs doses de produit fluide, ledit organe de distribution étant une pompe ou une valve adaptée à distribuer une dose à chaque actionnement.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique d'un dispositif de distribution nasale selon un mode de réalisation avantageux,
la figure 2 est une vue schématique de face d'un visage, montrant les points de repères utilisés par la caméra,
les figures 3 et 4 sont des vues schématiques du dispositif de la figure 1, en cours d'utilisation par l'utilisateur, montrant l'indicateur visuel du dispositif qui informe l'utilisateur en temps réel de l'orientation verticale du dispositif dans la narine,
les figures 5 et 6 sont des vues schématiques du dispositif de la figure 1, en cours d'utilisation par l'utilisateur, montrant l'orientation latérale du dispositif nasal dans la narine,
les figures 7 et 8 sont des vues schématiques du dispositif de la figure 1, en cours d'utilisation par l'utilisateur, montrant l'insertion du dispositif nasal dans la narine, et
les figures 9 et 10 sont des vues schématiques du dispositif de la figure 1, respectivement avant et après actionnement.

La présente invention concerne plus particulièrement un ensemble de distribution avec un dispositif nasal multidoses, c'est-à-dire comportant un réservoir contenant plusieurs doses.

Il est toutefois entendu que la présente invention ne se limite pas à ce type de dispositif, mais est au contraire applicable à tous les dispositifs de distribution de produit fluide et pulvérulents du type unidose, c'est-à-dire comportant un réservoir contenant une seule dose distribuée en un seul actionnement, bidose, c'est-à-dire comportant un réservoir contenant deux doses distribuées en deux actionnements successifs, ou multidoses, c'est-à-dire comportant un réservoir contenant plus de deux doses.

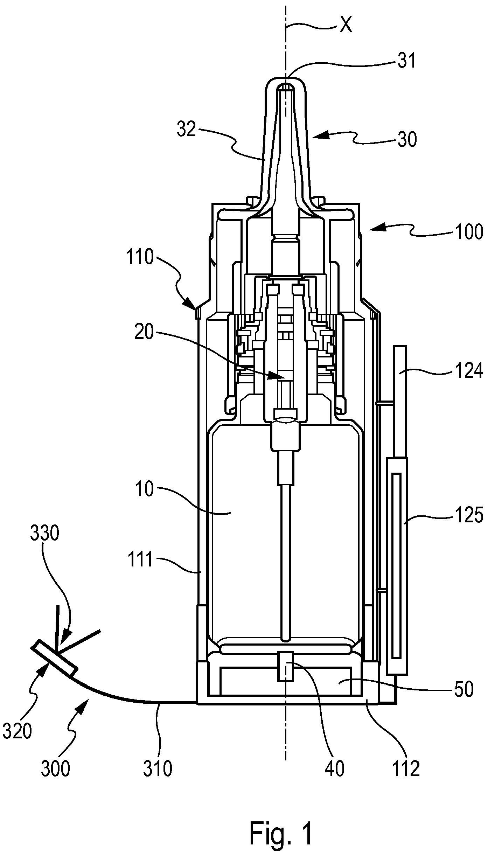

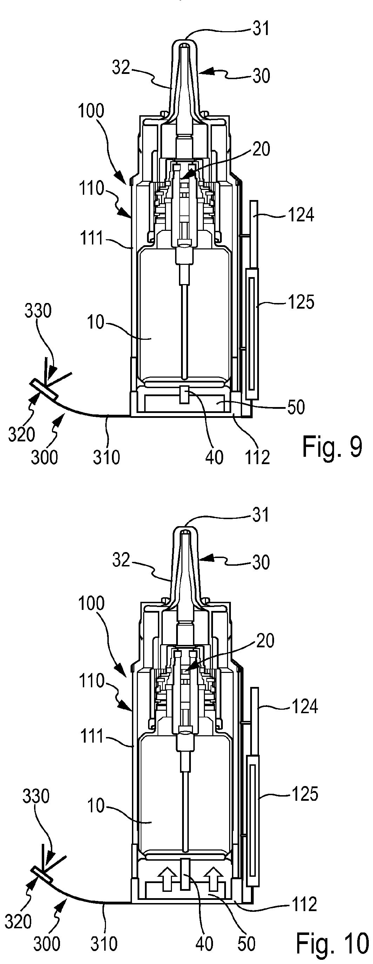

Dans la description ci-après, les termes "supérieur", "inférieur", "haut", "bas", "horizontal" et "vertical" se réfèrent à la position droite du dispositif représentée sur les figures 1, 9 et 10. Les termes "axial" et "radial" se réfèrent à l'axe central longitudinal X du dispositif, visible sur les figures 1 et 3 à 6.

Le dispositif nasal 100 donné à titre d'exemple est un multidose qui comporte un réservoir 10 contenant plusieurs doses de produit fluide, liquide ou pulvérulent.

Un organe de distribution 20, tel qu'une pompe ou une valve, est assemblé sur le réservoir 10.

Une tête de distribution 30 qui comporte un orifice de distribution 31 est assemblée sur ledit organe de distribution 20. La tête de distribution 30 comporte avantageusement un manchon creux 32, formant embout nasal, s'étendant axialement vers le haut et se terminant au niveau dudit orifice de distribution 31.

De préférence, le dispositif 100 comporte un corps 110. Celui-ci peut comporter un corps supérieur 111, disposé autour du réservoir 10 et de l'organe de distribution 20, et un corps inférieur 112, disposé sous ledit réservoir 10.

Le dispositif 100 comporte des moyens d'actionnement adaptés à déplacer le réservoir 10 par rapport à la tête de distribution 30, pour ainsi actionner l'organe de distribution 20.

Dans l'exemple représenté sur les figures, la tête de distribution 30 reste fixe par rapport au corps 110, et c'est le réservoir 10 qui est déplacé axialement vers le haut lors de l'actionnement par les moyens d'actionnement.

Avantageusement, l'actionnement du dispositif 100 est réalisé au moyen d'un élément poussoir 40 disposé dans le corps inférieur 112 et coopérant avec le fond du réservoir 10, pour déplacer ledit réservoir 10 axialement vers le haut par rapport à ladite tête de distribution 30.

Un moteur 50 peut être prévu pour actionner ledit élément poussoir 40 lorsque le dispositif est dans la bonne orientation dans la narine, comme cela sera expliqué plus en détails ci-après.

Avantageusement, le moteur 50 comporte une batterie rechargeable, par exemple au moyen d'une prise adaptée à cet effet.

Avantageusement, le corps inférieur 112 est amovible, notamment pour simplifier la recharge de la batterie.

Un objectif de l'invention est d'assurer un positionnement optimal du dispositif au moment même de l'actionnement du dispositif. Cela permet un meilleur taux de déposition du médicament dans la narine.

L'utilisateur peut éventuellement faire au préalable un test chez son spécialiste de santé afin de connaitre l'angle optimal adapté à sa morphologie, ce qui lui permet alors de configurer l'application associée. Eventuellement, on peut envisager l'utilisation d'un scanner ou d'une scintigraphie pour déterminer avec précision l'angle idéal.

Selon l'invention, le dispositif nasal 100 comporte une caméra 330 et un indicateur de positionnement 300.

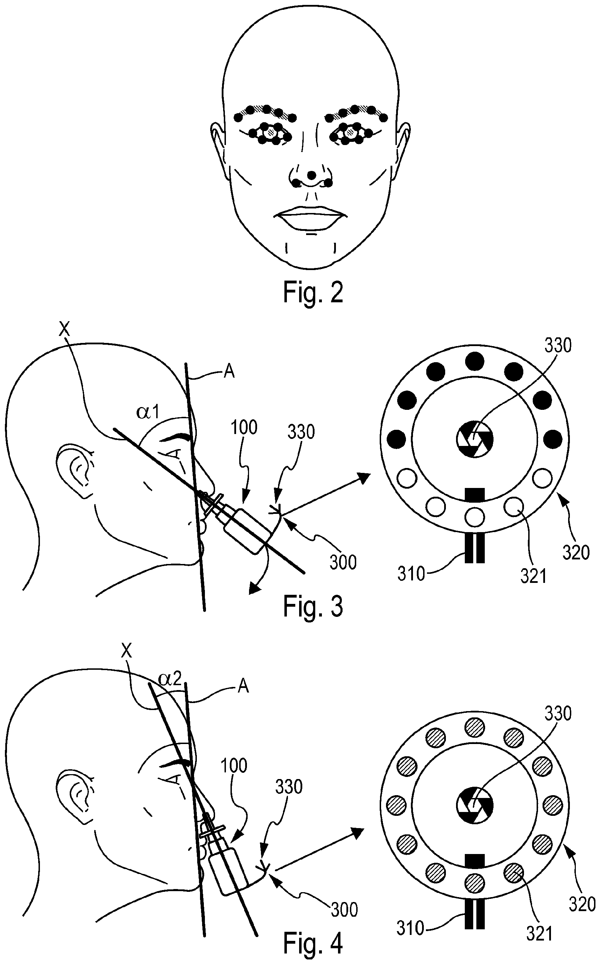

La caméra 330 permet de déterminer le positionnement spatial du dispositif nasal 100 par rapport au visage, et donc par rapport à la narine. La caméra 330 permet ainsi d'appliquer en temps réel des points de repères, notamment autour des yeux, des sourcils et du nez du patient, comme illustré sur la figure 2, et de calculer des distances entre ces différents points de repère permettant de connaître la position du dispositif nasal 100 par rapport au visage et donc la narine du patient.

Typiquement, la caméra 330 peut identifier plusieurs points de repère, par exemple vingt-cinq comme illustré sur la figure 2, qui montre trois repères sur le nez, six sur chaque oeil et cinq sur chaque sourcil. D'autres méthodes de reconnaissance faciale sont aussi envisageables.

La caméra détermine ainsi l'angle d'inclinaison verticale α de l'axe longitudinal X du dispositif nasal 100 par rapport à un axe A du visage défini par lesdits points de repère. L'orientation dudit axe A par rapport à la narine de l'utilisateur est toujours identique, quelle que soit la position de l'utilisateur. Cet angle α détermine l'inclinaison verticale du dispositif nasal 100, c'est-à-dire vers le haut ou le bas. La caméra 330 permet aussi de déterminer l'angle d'inclinaison latérale β entre les axes X et A, c'est-à-dire vers la gauche ou la droite. Avantageusement, la caméra 330 permet également de déterminer la profondeur d'insertion P.

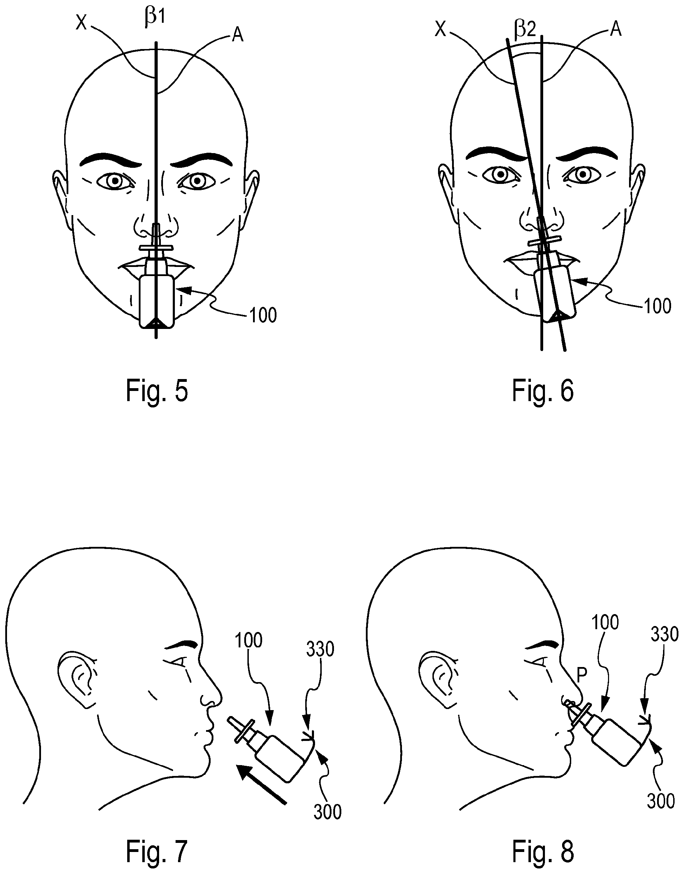

La figure 3 montre un angle vertical α₁ non optimal, ici environ 60°, alors que la figure 4 montre un angle vertical α₂ optimal, typiquement environ 30°. De même, la figure 5 montre un angle latéral β₁ non optimal, ici 0°, alors que la figure 6 montre un angle latéral β₂ optimal, typiquement environ 10°. Enfin, la figure 7 montre le dispositif nasal 100 non inséré, et la figure 8 montre le dispositif nasal inséré à une profondeur d'insertion P optimale.

La profondeur d'insertion P peut être déterminée par la caméra 330 en mesurant l'écart en pixels entre les deux yeux, cette distance augmentant au fur et à mesure que le dispositif nasal est inséré dans la narine, avec la caméra 330 qui se rapproche alors du visage.

L'indicateur de positionnement 300 permet de guider l'utilisateur vers un positionnement optimal du dispositif nasal 100 dans la narine. Cet indicateur 300 peut avantageusement être visuel, sonore et/ou tactile.

Les figures montrent un exemple d'un indicateur visuel.

Cet indicateur visuel 300 est disposé de préférence sur un bras 310 qui permet de déporter l'indicateur latéralement par rapport au dispositif nasal afin d'être visible de l'utilisateur même lorsque le dispositif nasal est inséré dans la narine. Ce bras 310 peut être par exemple monté pivotant sur la corps inférieur 112, comme montré sur les dessins. Bien entendu, d'autres mises en oeuvre sont possibles.

L'indicateur 300 comporte de préférence une tête d'indication lumineuse 320, pourvue d'une pluralités d'indicateurs lumineux 321, tels que des diodes électroluminescentes (LED). Avantageusement, ces LEDs sont disposées en cercle pour former une couronne de LEDs. Ces LEDs peuvent s'illuminer dans différentes couleurs, pour indiquer à l'utilisateur comment modifier la position du dispositif nasal dans la narine. D'autres mises en oeuvre sont possibles, par exemples quatre indicateurs lumineux sous forme de flèche, orientés dans les quatre directions cardinales.

De préférence, la caméra 330 est disposée au centre des indicateurs lumineux 321, comme illustré sur les figures 3 et 4.

Le dispositif nasal 100 comporte également un module électronique 125, tel qu'un circuit imprimé ou PCB, comportant un microprocesseur contenant le logiciel de traitement des informations fournies par la caméra et les moyens de commande du moteur d'actionnement 50.

Le module électronique 125 peut aussi intégrer une fonction de blocage du dispositif pendant un temps prédéterminable après chaque actionnement, notamment si le produit fluide contenu dans le réservoir 10 est un médicament dangereux, du type fentanyl.

Le module électronique peut avantageusement comporter en outre un module de communication, de préférence sans fil, avantageusement un module Bluetooth^{®}, pour une communication avec un appareil mobile distant, tel qu'un ordinateur, un smartphone ou une tablette, pour transmettre des informations, par exemple l'horodatage de la dernière prise de dose, celui de la prochaine dose, la date de péremption du produit fluide, etc. Le module de communication permet aussi de surveiller l'utilisation du dispositif nasal, et par exemple d'alerter un tiers en cas de non utilisation du dispositif dans les délais requis.

Avantageusement, le dispositif 100 peut comporter un écran 124, notamment tactile, adapté à afficher des informations visibles par l'utilisateur.

Il peut aussi comprendre un haut-parleur et/ou un élément vibrant, pour fournir une indication sonore et/ou tactile pour l'utilisateur, ce qui peut être utile pour des non-voyants par exemple. Cette indication sonore et/ou tactile peut être ajoutée à une indication visuelle, telle que celle décrite ci-dessus. Dans une autre variante, l'indicateur sonore et/ou tactile peut remplacer l'indicateur visuel.

Eventuellement, le dispositif nasal 100 peut comporter un capteur, tel qu'un accéléromètre, un gyroscope, un capteur de luminosité, un capteur infrarouge, un capteur d'humidité, un capteur de température, pour détecter quand le dispositif 100 est pris en main ou quand il est inséré dans une narine. Ce capteur n'est pas obligatoire, mais il peut être avantageux pour identifier le moment où le dispositif nasal 100 est effectivement inséré dans la narine. Il peut dans ce cas déclencher l'électronique pour la faire passer d'un mode veille à un mode actif.

Le fonctionnement de l'ensemble va être décrit ci-après en référence à l'exemple de réalisation représenté sur les figures.

Quelle que soit la position de l'utilisateur, debout, allongé ou assis, l'utilisateur déploie le bras indicateur 310 avant d'insérer le dispositif nasal 100 dans une narine.

Description des étapes de fonctionnement :
- Position repos : l'électronique 125 du dispositif est en veille ; l'application indique que le dispositif nasal 100 n'est pas en utilisation.
- Position prise en main : le déploiement du bras d'indicateur 310 active l'électronique 125 du dispositif; en variante, on peut utiliser le capteur optionnel pour activer l'électronique 125, comme expliqué précédemment, auquel cas cette activation ne se fait pas lors du déploiement du bras d'indicateur 310 mais lors de l'insertion du dispositif nasal 100 dans une narine.
- Position dispositif inséré dans la narine: la caméra 330 mesure par reconnaissance faciale l'angle d'inclinaison verticale α , l'angle d'inclinaison latérale β et la profondeur d'insertion P, et détermine ainsi la position du dispositif nasal 100 dans la narine; si le positionnement est hors de la zone optimale, une indication, telle que des LEDs de couleur rouge, s'affiche sur l'indicateur 300; selon les LEDs qui s'allument dans la couronne d'indication, l'utilisateur sait quelle direction est mauvaise et va donc déplacer le dispositif en conséquence pour améliorer la position; parallèlement, des indications sonores peuvent être émises, telles que par exemple "inclinez le dispositif vers le haut/bas" et/ou "tournez le dispositif vers votre gauche/droite" et/ou "insérez davantage"; de même, des vibrations du dispositif nasal peuvent aider l'utilisateur à améliorer le positionnement du dispositif nasal; lorsque l'utilisateur s'approche de l'orientation optimale, une indication différente, telle qu'une couleur différente pour les LEDs, peut s'afficher sur l'indicateur 300 et/ou les vibrations peuvent s'accélérer.
- Position optimale : lorsque l'utilisateur à positionné le dispositif nasal 100 dans la position optimale, toutes les LEDs s'affichent dans une couleur différente, par exemple en vert; les vibrations peuvent alors se transformer en un son continu; dans cette position optimale, l'électronique 125 actionne automatiquement le moteur 50 qui va pousser l'élément poussoir 40 et donc le réservoir 10 axialement vers le haut pour distribuer une dose de produit fluide ; parallèlement, une indication sonore peut être émise, telle que par exemple "actionnement du dispositif en cours"; ainsi, avant que la position optimale ne soit atteinte, le moteur 50 est inactif, et lorsque la position optimale est atteinte, le module électronique 125 enclenche le moteur 50 qui démarre l'administration de la dose de produit fluide.
- Après actionnement, l'écran 124 du dispositif 100, s'il est prévu, peut afficher l'horodatage de la dose qui a été émise, le nombre de doses restants à distribuer, l'horodatage de la prochaine dose, une indication de blocage du dispositif avec éventuellement un compte à rebours. D'autres indications sont aussi possibles, notamment si le dispositif 100 communique avec un appareil distant, tel qu'un ordinateur, une tablette ou un smartphone, pour transmettre des informations liées à l'utilisation du dispositif.

La présente invention procure ainsi de nombreux avantages :
- elle favorise un positionnement angulaire précis au moment de la distribution de dose, assurant ainsi un taux de déposition optimal du produit distribué dans la narine ;
- elle fournit une assistance "en temps réel" pour l'orientation ;
- elle assure une distribution automatique de la dose dès que la position optimale est atteinte, évitant ainsi tout risque de déplacement non souhaitable dû au geste d'actionnement; l'utilisateur n'a besoin que de tenir le dispositif nasal et de chercher la position optimale en fonction des indications de l'indicateur, l'actionnement de l'organe de distribution 20 étant géré automatiquement par la caméra 330, l'électronique 125 et le moteur 50;
- elle fournit une solution utilisable avec un grand nombre de dispositifs de distribution nasale du même type, et n'est donc pas limitée à l'exemple décrit ;
- elle a un impact mineur sur la conception et l'encombrement du dispositif, et reste de ce fait facilement transportable ;
- elle n'a pas d'impact sur le fonctionnement et les performances du dispositif, et ne modifie donc pas les performances du produit fluide distribué ;
- elle permet d'administrer facilement le produit fluide à un tiers ;
- elle permet l'utilisation en toute position, notamment debout, assis, allongé ;
- elle ne requiert aucun appareil supplémentaire, tel qu'un smartphone ou une oreillette, pour d'abord obtenir la position optimale dans la narine puis déclencher automatiquement la distribution de la dose dans cette position optimale ;
- elle permet un transfert de données aux médecins, spécialistes de santé, pharmaciens, réglementaire, assurances ;
- elle génère un auto-apprentissage : trouver l'angle optimal deviendra de plus en plus facile pour l'utilisateur, qui mémorisera inconsciemment la bonne position du dispositif par rapport à sa morphologie.

Bien entendu, la présente invention n'est pas limitée au dispositif multidoses décrit ci-dessus, mais s'applique à tout dispositif nasal, qu'il soit unidose, bidose ou multidoses.

La présente invention est notamment destinée au traitement de toute pathologie traitable par voie nasale nécessitant une orientation précise du dispositif nasal, tel que par exemple la maladie de Parkinson ou l'énurésie.

La présente invention a été décrite en référence à divers modes de réalisation, mais il est entendu qu'un homme du métier peut y apporter toutes modifications, sans sortir du cadre de la présente invention tel que défini par les revendications annexées.

## Revendications

1. Dispositif de distribution nasale de produit fluide (100), comportant un réservoir (10) contenant du produit fluide, un organe de distribution (20) pour distribuer une dose de produit fluide à chaque actionnement, et une tête de distribution (30) assemblée sur ledit organe de distribution (20), ladite tête de distribution (30) étant pourvue d'un orifice de distribution (31), **caractérisé en ce que** ledit dispositif nasal (100) comporte:
- une caméra (330) pour déterminer le positionnement spatial dudit dispositif nasal (100) dans une narine de l'utilisateur, ladite caméra (330) déterminant l'angle d'inclinaison verticale (α) et l'angle d'inclinaison latérale (β) entre l'axe longitudinal (X) dudit dispositif nasal (100) et un axe (A) du visage, ledit axe (A) étant identifié au moyen de plusieurs points de repère détectés par ladite caméra (330), notamment trois repères sur le nez, six sur chaque oeil et cinq sur chaque sourcil, l'orientation dudit axe (A) par rapport à ladite narine étant toujours identique quelle que soit la position de l'utilisateur,
- un indicateur de positionnement (300) pour guider l'utilisateur vers une orientation optimale du dispositif nasal (100) dans ladite narine, et
- un module électronique (125) et des moyens d'actionnement automatique (40; 50) adaptés à actionner automatiquement ledit dispositif nasal (100) lorsque ladite caméra (330) détermine que ledit dispositif nasal (100) est disposé dans une position optimale dans ladite narine.

2. Dispositif nasal (100) selon la revendication 1, comportant un corps (110), comprenant un corps supérieur (111) disposé autour dudit réservoir (10) et dudit organe de distribution (20), et un corps inférieur (112), disposé sous ledit réservoir (10).

3. Dispositif nasal (100) selon la revendication 2, dans lequel ledit corps inférieur (112) est amovible dudit corps supérieur (111).

4. Dispositif nasal (100) selon la revendication 2 ou 3, dans lequel ledit corps inférieur (112) contient lesdits moyens d'actionnement automatique, notamment un élément poussoir (40) et un moteur (50), adaptés lors de l'actionnement à déplacer ledit réservoir (10) axialement vers le haut par rapport à ladite tête de distribution (30), pour ainsi actionner ledit organe de distribution (20).

5. Dispositif nasal (100) selon l'une quelconque des revendications précédentes, dans lequel ledit indicateur de positionnement (300) est un indicateur visuel visible par l'utilisateur lorsque le dispositif nasal (100) est inséré dans ladite narine.

6. Dispositif nasal (100) selon la revendication 5, dans lequel ledit indicateur (300) comporte une tête d'indication lumineuse (320) pourvue d'une pluralités d'indicateurs lumineux (321), tels que des diodes électroluminescentes, adaptées à assister et guider l'utilisateur en temps réel pour améliorer le positionnement dudit dispositif nasal (100).

7. Dispositif nasal (100) selon la revendication 6, dans lequel ladite caméra (330) est disposée au centre desdits indicateurs lumineux (321).

8. Dispositif nasal (100) selon l'une quelconque des revendications précédentes, dans lequel ledit indicateur de positionnement (300) est un indicateur sonore et/ou tactile.

9. Dispositif nasal (100) selon l'une quelconque des revendications précédentes, dans lequel ladite caméra (330) et ledit indicateur (300) sont déportés radialement dudit dispositif nasal (100), en étant notamment formés sur un bras (310) solidaire, notamment de manière articulée, dudit dispositif nasal (100).

10. Dispositif nasal (100) selon l'une quelconque des revendications précédentes, dans lequel ladite caméra (330) détermine la profondeur d'insertion (P) du dispositif nasal (100) dans la narine, notamment par détermination de l'écart en pixels entre les deux yeux qui varie en fonction de la profondeur d'insertion.

11. Dispositif nasal (100) selon l'une quelconque des revendications précédentes, dans lequel ledit module électronique (125), tel qu'un circuit imprimé, comporte un microprocesseur contenant un logiciel de traitement des informations fournies par ladite caméra (330).

12. Dispositif nasal (100) selon l'une quelconque des revendications précédentes, comportant en outre un module de communication sans fil, avantageusement un module Bluetooth^{®}, pour une communication avec un appareil mobile distant.

13. Dispositif nasal (100) selon l'une quelconque des revendications précédentes, comprenant un écran (124) adapté à afficher des informations visibles par l'utilisateur.

14. Dispositif nasal (100) selon l'une quelconque des revendications précédentes, comprenant un haut-parleur et/ou un élément vibrant, pour fournir une indication sonore et/ou tactile pour guider l'utilisateur lors du positionnement du dispositif nasal (100) dans ladite narine.

15. Dispositif nasal (100) selon l'une quelconque des revendications précédentes, comportant un capteur, tel qu'un accéléromètre, un gyroscope, un capteur de luminosité, un capteur infrarouge, un capteur d'humidité, un capteur de température, pour détecter l'insertion dudit dispositif nasal (100) dans ladite narine.

16. Dispositif nasal (100) selon l'une quelconque des revendications précédentes, dans lequel ledit réservoir (10) contient plusieurs doses de produit fluide, ledit organe de distribution (20) étant une pompe ou une valve adaptée à distribuer une dose à chaque actionnement.

## Patentansprüche

1. Vorrichtung zur nasalen Verabreichung eines Fluidprodukts (100), aufweisend einen Vorratsbehälter (10), der Fluidprodukt enthält, ein Abgabeorgan (20) zur Abgabe einer Fluidproduktdosis bei jeder Betätigung, und einen Abgabekopf (30), auf dem Abgabeorgan (20) angebracht ist, wobei der Abgabekopf (30) mit einer Abgabeöffnung (31) versehen ist, **dadurch gekennzeichnet, dass** die nasale Vorrichtung (100) Folgendes aufweist:
- eine Kamera (330) zur Bestimmung der räumlichen Positionierung der nasalen Vorrichtung (100) in einem Nasenloch des Benutzers, wobei die Kamera (330) den Winkel vertikaler Neigung (α) und den Winkel seitlicher Neigung (β) zwischen der Längsachse (X) der nasalen Vorrichtung (100) und einer Achse (A) des Gesichts bestimmt, wobei die Achse (A) mittels mehrerer Bezugspunkte ermittelt wird, die von der Kamera (330) erfasst werden, insbesondere drei Bezugspunkte auf der Nase, sechs auf jedem Auge und fünf auf jeder Augenbraue, wobei die Orientierung der Achse (A) im Verhältnis zum Nasenloch immer identisch ist, unabhängig von der Position des Benutzers,
- einen Positionierungsanzeiger (300), um den Benutzer zu einer optimalen Orientierung der nasalen Vorrichtung (100) in dem Nasenloch zu führen, und
- ein elektronisches Modul (125) und Mittel zur automatischen Betätigung (40; 50), die geeignet sind, die nasale Vorrichtung (100) automatisch zu betätigen, wenn die Kamera (330) bestimmt, dass die nasale Vorrichtung (100) in einer optimalen Position im Nasenloch angeordnet ist.

2. Nasale Vorrichtung (100) nach Anspruch 1, aufweisend einen Körper (110), der einen Oberkörper (111), der um den Vorratsbehälter (10) und das Abgabeorgan (20) angeordnet ist, und einen Unterkörper (112), der unter dem Vorratsbehälter (10) angeordnet ist, umfasst.

3. Nasale Vorrichtung (100) nach Anspruch 2, wobei der Unterkörper (112) vom Oberkörper (111) abnehmbar ist.

4. Nasale Vorrichtung (100) nach Anspruch 2 oder 3, wobei der Unterkörper (112) die Mittel zur automatischen Betätigung enthält, insbesondere ein Stößel-Element (40) und einen Motor (50), die geeignet sind, bei der Betätigung den Vorratsbehälter (10) axial aufwärts im Verhältnis zum Abgabekopf (30) zu bewegen, um so das Abgabeorgan (20) zu betätigen.

5. Nasale Vorrichtung (100) nach einem der vorhergehenden Ansprüche, wobei der Positionierungsindikator (300) ein optischer Anzeiger ist, der für den Benutzer sichtbar ist, wenn die nasale Vorrichtung (100) in das Nasenloch eingeführt ist.

6. Nasale Vorrichtung (100) nach Anspruch 5, wobei der Anzeiger (300) einen Leuchtanzeigekopf (320) aufweist, der mit einer Mehrzahl von Leuchtanzeigern (321) wie etwa Leuchtdioden versehen ist, die geeignet sind, den Benutzer in Echtzeit zu unterstützen und zu führen, um die Positionierung der nasalen Vorrichtung (100) zu verbessern.

7. Nasale Vorrichtung (100) nach Anspruch 6, wobei die Kamera (330) in der Mitte der Leuchtanzeiger (321) angeordnet ist.

8. Nasale Vorrichtung (100) nach einem der vorhergehenden Ansprüche, wobei der Positionierungsanzeiger (300) ein akustischer und/oder haptischer Anzeiger ist.

9. Nasale Vorrichtung (100) nach einem der vorhergehenden Ansprüche, wobei die Kamera (330) und der Anzeiger (300) von der nasalen Vorrichtung (100) radial versetzt sind, und wobei sie insbesondere auf einem Arm (310) ausgebildet sind, der fest mit der nasalen Vorrichtung (100) verbunden, insbesondere an ihr angelenkt ist.

10. Nasale Vorrichtung (100) nach einem der vorhergehenden Ansprüche, wobei die Kamera (330) die Einführtiefe (P) der nasalen Vorrichtung (100) in das Nasenloch bestimmt, insbesondere durch Bestimmung des Abstands in Pixeln zwischen den zwei Augen, der je nach der Einführtiefe variiert.

11. Nasale Vorrichtung (100) nach einem der vorhergehenden Ansprüche, wobei das elektronische Modul (125), wie etwa eine Leiterplatte, einen Mikroprozessor aufweist, der eine Software zur Verarbeitung der Informationen enthält, die von der Kamera (330) bereitgestellt werden.

12. Nasale Vorrichtung (100) nach einem der vorhergehenden Ansprüche, ferner aufweisend ein drahtloses Kommunikationsmodul, vorteilhafterweise ein Bluetooth^{®}-Modul, für eine Kommunikation mit einem entfernten Mobilgerät.

13. Nasale Vorrichtung (100) nach einem der vorhergehenden Ansprüche, umfassend einen Bildschirm (124), der geeignet ist, Informationen anzuzeigen, die für den Benutzer sichtbar sind.

14. Nasale Vorrichtung (100) nach einem der vorhergehenden Ansprüche, umfassend einen Lautsprecher und/oder ein Vibrationselement zur Bereitstellung einer akustischen und/oder haptischen Anzeige, um den Benutzer bei der Positionierung der nasalen Vorrichtung (100) im Nasenloch zu führen.

15. Nasale Vorrichtung (100) nach einem der vorhergehenden Ansprüche, die einen Sensor wie etwa einen Beschleunigungsmesser, ein Gyroskop, einen Helligkeitssensor, einen Infrarotsensor, einen Feuchtesensor, einen Temperatursensor aufweist, um die Einführung der nasalen Vorrichtung (100) in das Nasenloch zu erkennen.

16. Nasale Vorrichtung (100) nach einem der vorhergehenden Ansprüche, wobei der Vorratsbehälter (10) mehrere Dosen Fluidprodukt enthält, wobei das Abgabeorgan (20) eine Pumpe und/oder ein Ventil ist, die bzw. das geeignet ist, bei jeder Betätigung eine Dosis abzugeben.

## Claims

1. A device (100) for dispensing a fluid product nasally, comprising a reservoir (10) containing fluid product; a dispensing means (20) for dispensing a dose of fluid product upon each actuation, and a dispensing head (30) which is assembled on said dispensing means (20), said dispensing head (30) being provided with a dispensing orifice (31), **characterized in that** the nasal device (100) comprises:
- a camera (330) for determining the spatial position of said nasal device (100) in a nostril of the user, said camera (330) determining the vertical angle of inclination (α) and the lateral angle of inclination (β) between the longitudinal axis (X) of said nasal device (100) and an axis (A) of the face, said axis (A) being identified by means of several reference points detected by said camera (330), in particular three reference points on the nose, six on each eye and five on each eyebrow, the orientation of said axis (A) with respect to said nostril always being identical irrespective of the position of the user,
- a positioning indicator (300) for guiding the user towards an optimal orientation of the nasal device (100) in said nostril, and
- an electronic module (125) and automatic actuation means (40; 50) adapted to automatically actuate said nasal device (100) when said camera (330) determines that said nasal device (100) is disposed in an optimal position in said nostril.

2. The nasal device (100) as claimed in claim 1, comprising a body (110) comprising an upper body (111) disposed around said reservoir (10) and said dispensing means (20), and a lower body (112) disposed under said reservoir (10).

3. The nasal device (100) as claimed in claim 2, in which said lower body (112) is removable from said upper body (111).

4. The nasal device (100) as claimed in claim 2 or claim 3, in which said lower body (112) contains said automatic actuation means, in particular a pusher element (40) and a motor (50), which are adapted, during actuation, to displace said reservoir (10) axially upwards with respect to said dispensing head (30) in order to actuate said dispensing means (20) thereby.

5. The nasal device (100) as claimed in any one of the preceding claims, in which said positioning indicator (300) is a visual indicator which is visible to the user when the nasal device (100) is inserted into said nostril.

6. The nasal device (100) as claimed in claim 5, in which said indicator (300) comprises a luminous indicator head (320) provided with a plurality of luminous indicators (321), such as light-emitting diodes, adapted to assist and guide the user in real time in order to improve the positioning of said nasal device (100).

7. The nasal device (100) as claimed in claim 6, in which said camera (330) is disposed at the centre of said luminous indicators (321).

8. The nasal device (100) as claimed in any one of the preceding claims, in which said positioning indicator (300) is an audible and/or tactile indicator.

9. The nasal device (100) as claimed in any one of the preceding claims, in which said camera (330) and said indicator (300) are radially offset from said nasal device (100), in particular being formed on an arm (310) which is integral with said nasal device (100), in particular in an articulated manner.

10. The nasal device (100) as claimed in any one of the preceding claims, in which said camera (330) determines the depth of insertion (P) of the nasal device (100) into the nostril, in particular by determining the distance in pixels between the two eyes, which varies as a function of the depth of insertion.

11. The nasal device (100) as claimed in any one of the preceding claims, in which said electronic module (125), such as a printed circuit, includes a microprocessor containing software for processing information provided by said camera (330).

12. The nasal device (100) as claimed in any one of the preceding claims further comprising a wireless communication module, advantageously a Bluetooth^{®} module, for communication with a remote mobile device.

13. The nasal device (100) as claimed in any one of the preceding claims, comprising a screen (124) which is adapted to display information that can be seen by the user.

14. The nasal device (100) as claimed in any one of the preceding claims, comprising a loudspeaker and/or a vibrating element for providing an audible and/or tactile indication to guide the user when positioning the nasal device (100) in said nostril.

15. The nasal device (100) as claimed in any one of the preceding claims, comprising a sensor, such as an accelerometer, a gyroscope, a brightness sensor, an infrared sensor, a humidity sensor, a temperature sensor, in order to detect the insertion of said nasal device (100) into said nostril.

16. The nasal device (100) as claimed in any one of the preceding claims, in which said reservoir (10) contains several doses of fluid product, said dispensing means (20) being a pump or a valve adapted to dispense one dose upon each actuation.
